# EUROPÄISCHE PATENTANMELDUNG

(11) **EP 2 898 878 A1**
(43) Veröffentlichungstag der Anmeldung: **29.07.2015**
(21) Anmeldenummer: 14152314.2
(22) Anmeldetag: 23.01.2014
(51) Int. Cl.: A61K 9/00, A61K 9/70, A61F 13/00

(54) **Klebeband enthaltend Beinwell**

(71) Anmelder: LTS LOHMANN Therapie-Systeme AG, 56626 Andernach (DE)
(72) Erfinder: Weiler, Erika, 56316 Niederhofen (DE); Laux, Wolfgang, 65582 Diez (DE)
(74) Vertreter: Held, Stephan

(57) **Zusammenfassung**

Die Erfindung betrifft ein Klebeband umfassend, eine wirkstoffundurchlässige Rückschicht, eine abziehbare Schutzschicht und eine wirkstoffhaltige Klebeschicht zwischen Rückschicht und Schutzschicht, dadurch gekennzeichnet dass, der Wirkstoff mindestens einen pharmazeutischen Wirkstoff, vorzugsweise einen nicht-verschreibungspflichtigen ("rezeptfreien") pharmazeutischen Wirkstoff und besonders bevorzugt Beinwell (Symphytum) umfasst, und dass die Klebeschicht auf mindestens einem medizinischen Haftkleber basiert.

## Beschreibung

Die vorliegende Erfindung betrifft ein wirkstoffhaltiges Klebeband enthaltend mindestens einen pharmazeutischen Wirkstoff, vorzugsweise einen nichtverschreibungspflichtigen ("rezeptfreien") pharmazeutischen Wirkstoff, und zwar insbesondere Beinwell (*Symphytum*). Die vorliegende Erfindung betrifft ferner ein derartiges Klebeband zur Behandlung verschiedener Krankheiten bzw. Schmerzzustände, insbesondere zur Behandlung und Prophylaxe von Erkrankungen und Verletzungen des Muskel-Skelett-Systems.

Aus der WO 2006/018340 A1 ist eine Kombination aus selbstklebendem Tape-Verband und wirkstoffhaltiger Hautauflage bekannt. Dabei ist die Hautauflage auf einem Trägermaterial, das mit einer Klebemasse beschichtet ist, aufgebracht. Diese Kombination dient zur Behandlung von Gelenkerkrankungen und zur Pflege der Haut im Gelenkbereich.

Die DE 10 2011 117 470 A1 offenbart ein topisch entzündungshemmendes Pflaster zur Beschleunigung der Gewebeheilung, sowie zur Schmerzminderung bei oberflächlichen Wunden oder geschlossenen Verletzungen. Als Wirkstoffe werden Amid- und Ester-Lokalanästhetika, wie z. B. Lidocain, eingesetzt.

Die US 2003/ 0091650 A1 beschreibt die Verwendung von Öl, gewonnen aus bestimmten Laufvögeln, wie Strauß, Emu, Nandu und Kasuar, als topisches Hilfsmittel für Agentien, die auf die Säugerhaut aufgetragen werden. Der Einsatz erstreckt sich dabei unter anderem auf Gele, Cremes, Salben, Lotionen, Emulsionen, Puder, Sprays, Patch-Systeme und selbstklebende Tapes.

Die EP 2 524 683 A1 beschreibt ein kinesiologisches Tape, das mindestens einen Formkörper aus Metall, Heilsteinen, Holz, Keramik und/oder Kombinationen davon enthält. Der Formkörper übt dabei Druck auf die Reflexpunkte der Haut aus, wodurch eine Akupressur-Wirkung des beklebten Körperteils erreicht wird. Dieser Formkörper kann zusätzlich einen Wirkstoff enthalten.

Die EP 2 468 227 B1 offenbart ein kinesiologisches Tape, aufgebaut aus einem dehnbaren Textilmaterial und einer Klebeschicht, die kleine Partikel, beispielsweise Holz, Sand oder Baumwolle, enthält. Die Klebschicht basiert auf Acrylaten.

Aufgabe der vorliegenden Erfindung ist es, ein wirkstoffhaltiges Klebeband zur Behandlung verschiedener Krankheits- bzw. Schmerzzustände, wie Migräne, Spannungskopfschmerzen, muskulären Schmerzzuständen, Gelenkschmerzen, Muskeischmerzen, Sehnenschmerzen, stumpfen Verletzungen, Rückenschmerzen, Kniearthrose, Prellungen, Zerrungen und/oder Verstauchungen, bereitzustellen.

Der Wirkstoff soll dabei topisch appliziert auf die betroffenen Hautareale einwirken. Das Klebeband sollte hinsichtlich der vorstehend genannten Krankheits- bzw. Schmerzzustände eine entzündungshemmende Wirkung aufweisen. Das Klebeband sollte ferner von der Haut gut toleriert werden und keine Hautirritationen hervorrufen, auch wenn es über einen längeren Zeitraum getragen wird. Das Klebeband soll schließlich eine ausreichende Dehnbarkeit, Flexibilität und Klebrigkeit aufweisen.

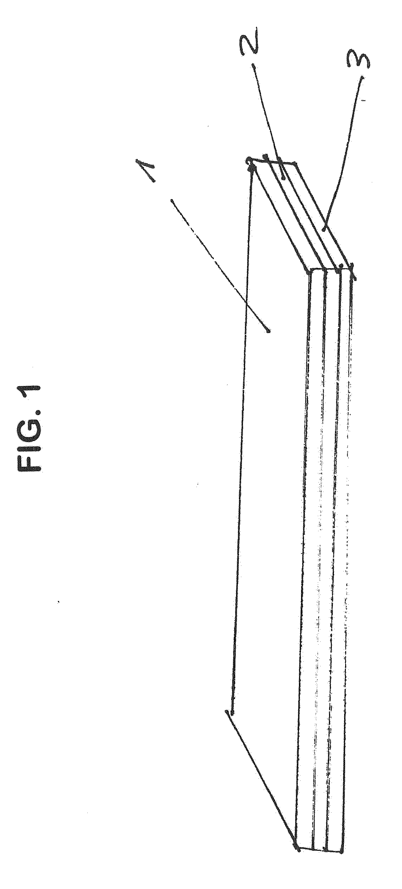

Obige Aufgabe wird durch ein Klebeband nach Anspruch 1 gelöst, dass eine wirkstoffundurchlässige Rückschicht (1), eine abziehbare Schutzschicht (3) und eine wirkstoffhaltige Klebeschicht (2) zwischen der Rückschicht und Schutzschicht umfasst, und dadurch gekennzeichnet ist, dass der Wirkstoff Beinwell (*Symphtum*) umfasst und dass die Klebeschicht auf mindestens einem medizinischen Haftkleber basiert (siehe Figur 1).

Das Klebeband kann weitere Schichten enthalten. So kann beispielsweise eine Sperrschicht vorhanden sein, die ein Abdiffundieren von flüchtigen Bestandteilen der wirkstoffhaltigen Klebeschicht und/oder ein Eindringen von Luftsauerstoff etc. verhindert bzw. vermindert. Eine solche Sperrschicht wäre beispielsweise zwischen wirkstoffhaltiger Klebeschicht (2) und wirkstoffundurchlässiger Rückschicht (1) angeordnet. Sie kann auch auf der wirkstoffhaltigen Klebeschicht abgewandten Seite der wirkstoffundurchlässigen Rückschicht (1) angebracht sein.

Das Klebeband kann auch eine weitere Schicht enthalten, die als Reservoir für den mindestens einen Wirkstoff dient und/oder mindestens einen pharmazeutisch nicht wirksamen Hilfsstoff enthält.

Das Klebeband kann auch eine Kontrollschicht enthalten, die die Freisetzung des mindestens einen Wirkstoffs kontrolliert und so eine zeitlich längere Wirkungsdauer gewährleistet.

Bei Beinwell (*Symphytum*) handelt es sich um eine Pflanzengattung, die zur Familie der Raubblattgewächse (Boraginaceae) zählt und der über 40 verschiedene Arten zuzurechnen sind. Frühere Bezeichnungen lauten Wallwurz oder Beinwurz, die von der dem Beinwell zugeschriebenen Eigenschaft Knochenbrüche und (offene) Wunden zu heilen herrühren. Beinwell wird daher in der Naturheilkunde und in der fernöstlichen Medizin bereits seit geraumer Zeit als Heilkraut verwendet, da Beinwell eine heilende Wirkung bei Verletzungen von Sehnen und Bändern, bei Prellungen, Zerrungen und Verstauchungen zugeschrieben wird. Seine schmerzstillende, entzündungshemmende und abschwellende Wirkung ist auf die Inhaltsstoffe Rosmarinsäure ((E,R)-3-(3,4-Dihydroxy-phenyl)-acrylsäure-1-carboxy-2-(3,4-dihydroxy-phenyl)-ethylester) und Allantoin (N-(2,5-Dioxo-4-imidazolidinyl)-harnstoff) zurückzuführen. Allantoin, ein primäres Endprodukt des Purinbasen-Abbaus, das auch in zahlreichen Kosmetika Verwendung findet, trägt zu einer Beschleunigung des Zellaufbaus und der

Zellbildung bei und ist in der Beinwellwurzel mit etwa 0,6 bis 4,7 % (Dennis *et al*., 1987) enthalten. Die Rosmarinsäure trägt zu der angesprochenen Heilwirkung durch ihre antiviralen, antibakteriellen und antiflammatorischen Eigenschaften bei und konnte in der Beinwellwurzel bislang mit bis zu 0,2 % nachgewiesen werden.

Unter einem medizinischen Haftkleber wird ein hochviskoser, elastischer Klebstoff verstanden, der allein durch seine Molekulargewichtsverteilung, ohne den Zusatz von Klebrigmachern, selbstklebend ist und über ein sehr geringes allergenes Potential verfügt und somit die Haut nicht reizt. In abgebundener Form bildet ein Haftkleber einen trockenen Film, der bei Raumtemperatur permanent klebrig ist und klebfähig bleibt. Die Klebung erfolgt durch leichten Anpressdruck sofort auf fast allen Substraten.

Das erfindungsgemäße Klebeband mit dem Wirkstoff Beinwell und mindestens einem medizinischen Haftkleber als Basis für die Klebeschicht ist in der Lage ausreichend große, wirksame Mengen des Wirkstoffes Beinwell aufzunehmen. Es besteht eine gute Kompatibilität zwischen dem verwendeten, mindestens einem medizinischen Haftkleber und dem Wirkstoff Beinwell.

Ferner haftet das erfindungsgemäße Klebeband ausreichend gut auf der Haut, während die Haut jedoch nicht irritiert wird. Das erfindungsgemäße Klebeband kann leicht auf die betroffenen schmerzenden Hautareale appliziert werden.

Das erfindungsgemäße Klebeband führt zu dem synergistischen Vorteil, dass die vorstehend genannte Behandlung und/oder Prophylaxe verschiedener Krankheits-bzw. Schmerzzustände sowohl mit dem Klebeband an sich (durch das "Tapen") als auch mit dem darin enthaltenden, pharmazeutischen Wirkstoff, vorzugsweise einer entzündungshemmenden Substanz, insbesondere Beinwell, behandelt werden können. Dies führt dazu, dass die Behandlungsdauer vermindert wird. Auch wird durch die Kombination dieser beiden Prinzipien eine Tiefenwirkung auf Muskel- und Skelettsystem erreicht.

Die Verabreichung des Wirkstoffs erfolgt topisch. Darunter versteht man die lokale Verabreichung. Der Wirkungsort ist weitgehend identisch mit dem Ort der Verabreichung. Eine Verteilung des Wirkstoffes im Körper über die Blutbahnen ist nicht vorgesehen. Dem steht eine systemische Verabreichung gegenüber, bei der ein Wirk- oder Arzneistoff in Form einer Tablette oder eines transdermalen Therapiesystems verabreicht wird. Der Wirkstoff wird dabei zunächst in die Blutbahn geschleust und dann im gesamten Körper verteilt. Der Verabreichungsort ist bei derartigen Anwendungen mit dem Wirkungsort nicht identisch. Somit eröffnet das erfindungsgemäße Klebeband die Möglichkeit eine topische Therapie durchführen zu können und bietet die Vorteile, dass der mindestens eine Wirkstoff gezielt lokal an dem benötigen Hautareal eingesetzt werden kann. Unverletzte oder nicht schmerzende Hautpartien, oder gar der gesamte Körper, wie bei den Darreichungsformen Tablette oder transdermales Therapiesystem, werden nicht belastet.

In einer bevorzugten Ausführungsform wird Beinwell aus dem Pflanzenextrakt der Wurzel der Beinwellpflanze gewonnen.

Gemäß einer bevorzugten Ausführungsform ist das erfindungsgemäße Klebeband dadurch gekennzeichnet dass, die Klebeschicht 0,1 bis 40 Gew.-%, bevorzugt 0,5 bis 30 Gew.-% und besonders bevorzugt 1 bis 20 Gew.-% des Wirkstoffes Beinwell enthält.

In einer bevorzugten Ausführungsform enthält das erfindungsgemäße Klebeband kein Öl der Laufvögel Strauß, Emu, Nandu und Kasuar in der wirkstoffhaltigen Klebeschicht.

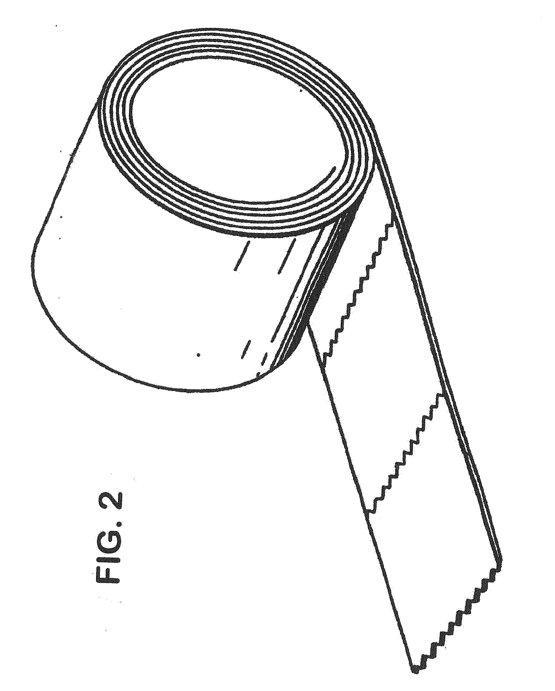

Vorzugsweise handelt es sich bei dem erfindungsgemäßen Klebeband um ein Tape. Unter einem Tape wird vorliegend ein elastisches, selbstklebendes Band in Streifenform verstanden, das heißt ein Abschnitt des erfindungsgemäßen Klebebandes mit definierter Länge und Breite. Die Dimensionen dieser Abschnitte können auf einem "endlosen" Band (in Rollenform) vorgeschnitten oder durch Sollbruchlinien oder Perforationen vorgegeben sein. Ein solches Band ist der Figur 2 zu entnehmen.

Das Tape wird bei Anwendung auf die betroffenen Hautpartien passend zum Verlauf der zu unterstützenden Muskeln und Sehnen aufgebracht. Bei dieser als "Taping" bezeichneten Technik kann ein Tape durch den sogenannten Hautfalteneffekt wirken. Darunter versteht man, dass nach Aufbringung des Tapes die beklebte Haut zusammen mit dem Tape bei Zurückführung in den Ruhezustand des Muskels kleine, wellenförmige Hautfalten ausbildet. Dies begünstigt ein Anheben der Haut, das darunter liegende Hautgewebe vergrößert sich, was sich positiv auf den Blut- und Lymphfluss auswirkt und einen gewissen Massageeffekt zeigt.

Gemäß einer bevorzugten Ausführungsform ist das erfindungsgemäße Klebeband bi-elastisch. Unter Bi-Elastizität wird hier die Fähigkeit des Klebebandes sich in zwei verschiedene Richtungen, vorzugsweise in Längs- und Querrichtung, bezogen auf den Ausgangszustand des Materials, zu dehnen, ohne das die Grundform verloren geht, verstanden. Eine dauerhafte Verformung des gedehnten Materials tritt nicht auf. Die Elastizität wird mit Hilfe der Dehnung, die als dimensionslose Zahl oder mit 100 multipliziert als Prozentwert angegeben wird, bewertet. Beim erfindungsgemäßen Klebeband beträgt die Dehnung in zwei verschiedene Richtungen, bevorzugt in Längs- und Querrichtung, vorzugsweise 0 bis 100 %, besonders bevorzugt 10 bis 50 % und ganz besonders bevorzugt 15 bis 30 %, bezogen auf die ursprünglichen Abmessungen des Klebebandes. Die Elastizität wird gemäß ISO 13934-1 vom 10. April 2013 bestimmt.

Gemäß einer weiteren bevorzugten Ausführungsform ist das erfindungsgemäße Klebeband längs-elastisch. Unter Längselastizität versteht man die Fähigkeit eines Materials sich in eine Richtung, der Längsrichtung, bezogen auf den Ausgangszustand des Materials, zu dehnen, ohne das die Grundform verloren geht. Eine dauerhafte Verformung des gedehnten Materials liegt auch hier nicht vor. Die Elastizität wird mit Hilfe der Dehnung, wie vorstehend beschrieben, bewertet. Beim erfindungsgemäßen Klebeband beträgt die Dehnung in Längsrichtung vorzugsweise 0 bis 100 %, besonders bevorzugt 10 bis 50 % und ganz besonders bevorzugt 15 bis 30 %, bezogen auf die ursprünglichen Abmessungen des Klebebandes. Die Bestimmung der Dehnung erfolgt wie oben angegeben. Ein längselastisches Klebeband bietet den Vorteil einer vereinfachten Herstellung.

Wie den obigen Ausführungen zu entnehmen ist, ist die Bi-Elastizität dahingehend von der Längselastizität zu trennen, dass Erstere eine Elastizität in zwei und Letztere eine Elastizität in ausschließlich eine Richtung, der Längsrichtung, aufweist.

Besonders bevorzugt ist der mindestens eine medizinische Haftkleber aus der Gruppe bestehend aus Poly(meth)acrylaten, Siliconen und/oder Polyisobutylenen ausgewählt, wobei Polysiobutylene (in Verbindung mit Klebharzen) und Poly(meth)acrylate aufgrund niedriger Rohstoffkosten ganz besonders bevorzugt sind.

Basispolymere weiterer geeigneter Haftklebstoffe sind Naturkautschuk und Synthesekautschuke, Polyester, Polychloroprene, Polyvinylether und Polyurethane, die in Kombination mit Zusätzen wie Harzen, Weichmachern und/oder Antioxidantien eingesetzt werden. Haftklebstoffe können als wässrige Systeme (Dispersionsklebstoffe), als lösemittelbasierende Systeme (Lösemittelklebstoffe) und als Schmelzklebstoff-Systeme formuliert werden. Die Viskosität des Poly(meth)acrylates in dem mindestens einen medizinischen Haftkleber liegt vorzugsweise im Bereich von 500 bis 25000, besonders bevorzugt im Bereich von 1000 bis 20000 und ganz besonders bevorzugt im Bereich von 1500 bis 12000 mPa·s bei 25 °C.

Gemäß einer bevorzugten Ausführungsform ist der mindestens eine medizinische Haftkleber des erfindungsgemäßen Klebebandes ein Polyacrylat auf Basis von Acrylsäure, Butylacrylat, 2-Ethylhexylacrylat und Vinylacetat oder auf Basis von Acrylsäure, 2- Ethylhexylacrylat und Methylacrylat.

In einer anderen bevorzugten Ausführungsform ist der mindestens eine medizinische Haftkleber des erfindungsgemäßen Klebebandes aus einer zweiphasigen Klebematrix, die hydrophile Polyacrylate und lipophile Hilfsstoffe umfasst, aufgebaut. Der Wirkstoff kann dabei in einer oder beiden Komponenten einer solchen, zweiphasigen Klebematrix enthalten sein.

Vorzugsweise ist das erfindungsgemäße Klebeband dadurch gekennzeichnet dass, der mindestens eine medizinische Haftkleber mehr als 60 Gew.-% Polyacrylat, bevorzugt mehr als 65 Gew.-% und besonders bevorzugt mehr als 70 Gew.-% Polyacrylat enthält.

Vorzugsweise ist das erfindungsgemäße Klebeband streifenförmig, auf eine Rolle zur Lagerung aufgerollt und hat vorzugsweise eine maximale Breite von bis zu 20 cm, bevorzugt bis zu 15 cm, besonders bevorzugt bis zu 10 cm und ganz besonders bevorzugt bis zu 5 cm. Die Länge der (individuellen) Tapes kann an den Ort der vorgesehenen Behandlung angepasst werden. Ein Tape kann daher bis zu einem Meter lang sein, vorzugsweise bis zu 50 cm, besonders bevorzugt bis zu 30 cm.

Bei der abziehbaren Schutzschicht des erfindungsgemäßen Klebebandes handelt es sich vorzugsweise um eine einseitig silikonierte Folie oder ein silikoniertes Papier, das vor der Aufbringung des erfindungsgemäßen Klebebandes auf die Haut abgezogen wird. Diese Schicht schützt die darunter liegende Klebeschicht vor übermäßiger Lichteinstrahlung und somit Austrocknung der wirkstoffhaltigen Klebeschicht. In einer bevorzugten Ausführungsform der Schutzschicht besteht diese aus einer einseitig silikonierten Polyethylenterephthalat-Schicht mit vorzugsweise einer Dicke von 15 bis 100 µm. Der Wirkstoff kann somit ideal im erfindungsgemäßen Klebeband über eine längeren Zeitraum von bis zu 36 Monaten, bevorzugt bis zu 18 Monate und besonders bevorzugt bis zu 12 Monate gelagert werden.

Die wirkstoffundurchlässige Rückschicht des Klebebandes ist vorzugsweise aus Textilmaterialien auf Basis von Baumwolle, Polyester oder einem anderen faserförmigen Polymermaterial aufgebaut.

Die wirkstoffundurchlässige Rückschicht kann auch aus einem folienförmigen Material aufgebaut sein. Hierfür kommen insbesondere Polyurethan und/oder Polyvinylacetat in Frage.

Unter "Wirkstoffundurchlässigkeit" im Zusammenhang mit der Rückschicht ist die Eigenschaft zu verstehen, ein unerwünschtes Austreten des mindestens einen pharmazeutischen Wirkstoffs durch die der Haut abgewandten Seite des Klebebands (bzw. des Tapes) während der Lagerung und während der Anwendung zu verhindern. Aufgrund der hohen Elastizität dieser Schicht und der unbekannten mechanischen Beanspruchung während der Anwendung kann aber ein marginaler Wirkstoffverlust nicht immer vollständig ausgeschlossen werden.

Die wirkstoffundurchlässige Rückschicht weist selbstverständlich die Elastizitätseigenschaften auf, die für das wirkstoffhaltige Klebeband kennzeichnend sind.

Das wirkstoffhaltige Klebeband enthält mindestens einen pharmazeutischen Wirkstoff, vorzugsweise einen nicht-verschreibungspflichtigen ("rezeptfreien") pharmazeutischen Wirkstoff. Als am besten geeigneter Wirkstoff kommt insbesondere Beinwell in Frage.

Auch können Stoffe im wirkstoffhaltigen Klebeband enthalten sein, die keine pharmazeutische Wirkung aufweisen, aber das Eindringen des mindestens einen Wirkstoffs, vorzugsweise Beinwell, in die Haut verbessern (so genannte "Penetrationsverstärker"). Hierzu zählen Alkohole wie Ethanol, Isopropanol und Glycole, Fettsäuren und Fettsäureester, Azone, Sulfoxide, Pyrrolidone, etherische Öle, Terpene, Terpenoide, Oxazolidinone und Harnstoff. Derartige Penetrationsverstärker sind dem Fachmann bekannt.

Vorzugsweise kann das erfindungsgemäße Klebeband neben Beinwell noch weitere Wirkstoffe enthalten.

Hierfür kommen in Frage: entzündungshemmende Stoffe; Stoffe, die die Durchblutung fördern; physiologische Kühlmittel; Schmerzmittel, insbesondere nicht-steroidale Analgetika; fiebersenkende Mittel.

Bevorzugte weitere Wirkstoffe sind (-)-Menthol, Ibuprofen, Diclofenac, Acetylsalicylsäure, Methylnicotinat und/oder Lidocain, und/oder Mineralien. (-)-Menthol führt zu einem angenehmen Kältegefühl auf der Haut. Dies kann auf eine Reizung der kälteempfindlichen Nerven und eine Oberflächen-Anästhesierung zurückgeführt werden. Dieser Effekt wirkt sich besonders vorteilhaft zur Behandlung von Migräne aus. Ibuprofen (2-(4-Isobutylphenyl)propionsäure), Acetylsalicylsäure und Diclofenac wirken durch die nichtselektive Hemmung der Cyclooxygenasen I und II schmerzstillend, entzündungshemmend und fiebersenkend. Methylnikotinat fördert die Durchblutung. Das Lokalanästhetikum Lidocain blockiert spannungsabhängige Natrium-Kanäle in den Zellmembrannervenzellen. Eine daraus resultierende Blockierung der Erregungsweiterleitung über die Nervenzellen verhindert die Weitergabe der Schmerzinformation an das Gehirn. Mineralien können dazu beitragen, die Ausscheidung von Giftstoffen und Schlacken über die Haut zu begünstigen.

In einer weiteren bevorzugten Ausführungsform enthält das erfindungsgemäße Klebeband keine Inhaltsstoffe wie Methyl-, Ethyl-, Propyl-, Butyl-, und 2-Methylpropyl-4-hydroxybenzoate (Parabene), Alkohole und Erdnussöl, die allergische Reaktionen, Hautreizungen, Hautentzündungen, Hauttrockenheit und lokale Hautreaktionen (z. B. Kontaktdermatitis) verursachen können.

Das erfindungsgemäße Klebeband kann als therapeutisches Mittel eingesetzt werden.

Vorzugsweise dient das erfindungsgemäße Klebeband zur Prophylaxe und Behandlung von Migräne, Spannungskopfschmerzen und Kombinationen aus Migräne und Spannungskopfschmerzen. Das erfindungsgemäße Klebeband wird auch zur Prophylaxe und Behandlung von Gelenkschmerzen, Muskelschmerzen, Sehnenschmerzen, stumpfen Verletzungen, Rückenschmerzen, Kniearthrosen, Prellungen, Zerrungen und/oder Verstauchungen eingesetzt. Hierzu zählen auch Schiefhals, Tennisarm, Golfarm, Mausarm, Wadenkrampf.

Ein weiterer bevorzugter Aspekt der beschriebenen Erfindung schließt die Verwendung des erfindungsgemäßen Klebebandes zur Prophylaxe und Behandlung von Migräne, Spannungskopfschmerzen und Kombinationen aus Migräne und Spannungskopfschmerzen ein. Das erfindungsgemäße Klebeband wird auch verwendet zur Prophylaxe und Behandlung von Gelenkschmerzen, Muskelschmerzen, Sehnenschmerzen, stumpfen Verletzungen, Rückenschmerzen, Kniearthrosen, Prellungen, Zerrungen und/oder Verstauchungen.

Das erfindungsgemäße Klebeband kann über einen Zeitraum von mindestens einem halben Tag getragen werden. Vorzugsweise beträgt die Tragedauer mindestens einen Tag, maximal 7 bis 10 Tage. Ein idealer Anwendungszeitraum beträgt 2 bis 3 Tage.

### Beispiel 1:

### Herstellung eines Beinwell enthaltendes Klebebands:

500 g Beinwell-Wurzelextrakt werden zu 9 kg DuroTak 2287 (50%-ige Lösung in Ethylacetat) gegeben und bis zur Homogenität gerührt. Diese Masse wird in einer Schichtdicke von 120 µm auf die silikonisierte Seite eines Trägerpapiers ausgestrichen und getrocknet. Die so entstandene haftklebende Seite wird nun unverzüglich mit einem bielastischen Gewebe aus Polyester abgedeckt. Durch Längsschneiden erhält man Streifen von 7 cm Breite, die zu einem "endlosen" Band aufgerollt werden. Die wirkstoffhaltige Klebeschicht enthält etwa 10 Gew.-% Beinwell.

### Beispiel 2:

### Verwendung eines Beinwell enthaltenden Klebebandes:

Zwei etwa 20 cm lange Streifen eines Beinwell enthaltenden Klebebandes (aus Beispiel 1) werden längs der beiden trapezförmigen Muskeln (musculus trapezius) im Nackenbereich einer Patientin mit Migräne und damit zeitgleich auftretenden Spannungskopfschmerz appliziert und für drei Tage getragen.

Es zeigt sich, dass durch diese Anwendung ein Auftreten von Migräne verhindert und die Intensität eines Anfalls von Spannungskopfschmerz deutlich verringert wird.

## Patentansprüche

1. Klebeband umfassend,
eine wirkstoffundurchlässige Rückschicht (1),
eine abziehbare Schutzschicht (3) und
eine wirkstoffhaltige Klebeschicht (2) zwischen Rückschicht und Schutzschicht, **dadurch gekennzeichnet dass**, der Wirkstoff mindestens einen pharmazeutischen Wirkstoff, vorzugsweise einen nichtverschreibungspflichtigen ("rezeptfreien") pharmazeutischen Wirkstoff und besonders bevorzugt Beinwell (*Symphytum*) umfasst, und dass die Klebeschicht auf mindestens einem medizinischen Haftkleber basiert.

2. Klebeband nach Anspruch 1, **dadurch gekennzeichnet dass**, die Klebeschicht (2) 0,1 bis 40 Gew.-%, bevorzugt 0,5 bis 30 Gew.-% und besonders bevorzugt 1 bis 20 Gew.-% Beinwell enthält.

3. Klebeband nach Anspruch 1 und 2, wobei das Klebeband ein Tape ist.

4. Klebeband nach mindestens einem der vorangehenden Ansprüche, **dadurch gekennzeichnet dass**, das Klebeband bi-elastisch oder längs-elastisch ist.

5. Klebeband nach mindestens einem der vorangehenden Ansprüche, **dadurch gekennzeichnet dass**, der mindestens eine medizinische Haftkleber aus der Gruppe bestehend aus Poly(meth)acrylaten, Siliconen und/oder Polyisobutylenen, bevorzugt aus der Gruppe bestehend aus Poly(meth)acrylaten und Polyisobutylenen, vorzugsweise in Verbindung mit Klebeharzen, ausgewählt ist.

6. Klebeband nach mindestens einem der vorangehenden Ansprüche, **dadurch gekennzeichnet dass**, der mindestens eine medizinische Haftkleber Polymere auf der Basis von Naturkautschuken, Synthesekautschuken, Polyestern, Polychloroprenen, Polyvinylethern und Polyurethanen, die in Kombination mit Zusätzen wie Harzen, Weichmachern und/oder Antioxidantien eingesetzt werden, enthält.

7. Klebeband nach mindestens einem der vorangehenden Ansprüche, **dadurch gekennzeichnet dass**, der mindestens eine medizinische Haftkleber ein Polyacrylat auf Basis von Acrylsäure, Butylacrylat, 2-Ethylhexylacrylat und Vinylacetat oder auf Basis von Acrylsäure, 2-Ethylhexylacrylat und Methylacrylat ist.

8. Klebeband nach mindestens einem der vorangehenden Ansprüche, **dadurch gekennzeichnet dass**, der mindestens eine medizinische Haftkleber aus einer zweiphasigen Klebematrix, die hydrophile Polyacrylate und lipophile Hilfsstoffe umfasst, aufgebaut ist, wobei der mindestens eine Wirkstoff in einer oder beiden Komponenten der zweiphasigen Klebematrix enthalten ist.

9. Klebeband nach mindestens einem der vorangehenden Ansprüche, **dadurch gekennzeichnet dass**, der mindestens eine medizinische Haftkleber mehr als 60 Gew.-% Polyacrylat, bevorzugt mehr als 65 Gew.-% und besonders bevorzugt mehr als 70 Gew.-% Polyacrylat enthält.

10. Klebeband nach mindestens einem der vorangehenden Ansprüche, **dadurch gekennzeichnet dass**, die wirkstoffundurchlässige Rückschicht (1) aus einem folienförmigen Material, bevorzugt aus einem folienförmigen Material auf Basis von Polyurethan und/oder Polyvinylacetat aufgebaut ist.

11. Klebeband nach mindestens einem der vorangehenden Ansprüche, **dadurch gekennzeichnet dass**, die Klebeschicht weitere Wirkstoffe, ausgewählt aus der Gruppe bestehend aus entzündungshemmenden Stoffen, Stoffen, die die Durchblutung fördern, physiologischen Kühlmitteln, Schmerzmitteln, nichtsteroidalen Analgetika und/oder fibersenkenden Mitteln, bevorzugt (-)-Menthol, Ibuprofen, Diclofenac, Acetylsalicylsäure, Methylnicotinat und/oder Lidocain und/oder Mineralien umfasst.

12. Klebeband nach mindestens einem der vorangehenden Ansprüche, **dadurch gekennzeichnet dass**, die Klebeschicht weitere Stoffe mit nicht-pharmazeutischer Wirkung ausgewählt aus der Gruppe bestehend aus Alkoholen, bevorzugt Ethanol, Isopropanol und Glycole, Fettsäuren und Fettsäureestern, Azonen, Sulfoxiden, Pyrrolidonen, etherischen Ölen, Terpenen, Terpenoiden, Oxazolidonen und Harnstoff umfasst.

13. Klebeband nach mindestens einem der vorangehenden Ansprüche als therapeutisches Mittel.

14. Klebeband nach mindestens einem der vorangehenden Ansprüche zur Prophylaxe und Behandlung von Migräne, Spannungskopfschmerzen, Kombinationen aus Migräne und Spannungskopfschmerzen, Gelenkschmerzen, Muskelschmerzen, Sehnenschmerzen, stumpfen Verletzungen, Rückenschmerzen, Kniearthrosen, Prellungen, Zerrungen und/oder Verstauchungen sowie zur Prophylaxe und Behandlung von Schiefhals, Tennisarm, Golfarm, Mausarm und/oder Wadenkrämpfen.

15. Verwendung eines Klebebandes nach mindestens einem der vorangehenden Ansprüche zur Prophylaxe und Behandlung von Migräne, Spannungskopfschmerzen, Kombinationen aus Migräne und Spannungskopfschmerzen, Gelenkschmerzen, Muskelschmerzen, Sehnenschmerzen, stumpfen Verletzungen, Rückenschmerzen, Kniearthrosen, Prellungen, Zerrungen und/oder Verstauchungen sowie zur Prophylaxe und Behandlung von Schiefhals, Tennisarm, Golfarm, Mausarm und/oder Wadenkrämpfen.
